# NOUVEAU FASCICULE DE BREVET EUROPEEN

(11) **EP 1 004 291 B2**
(45) Date de publication et mention de la décision concernant l'opposition: **21.01.2009**
(45) Mention de la délivrance du brevet: 19.10.2005
(21) Numéro de dépôt: 99121005.5
(22) Date de dépôt: 21.10.1999
(51) Int. Cl.: A61Q 5/00, A61Q 5/02, A61Q 5/12, A61Q 19/00, A61Q 19/10, A61K 8/84, A61K 8/39, A61K 8/891

(54) **Composition conditionnante et détergente et utilisation**
Konditionierungs- und Reinigungsmittelzusammensetzung, deren Verwendung
Conditioning and detergent composition, its use

(30) Priorité: 12.11.1998 FR 9814217
(43) Date de publication de la demande: 31.05.2000
(73) Titulaire: L'ORÉAL, 75008 Paris (FR)
(72) Inventeur: Garnier, Nathalie, Springfield, NJ 07081 (US)
(74) Mandataire: Martin-Charbonneau, Virginie

(56) Documents cités:
- EP-A- 0 122 324
- EP-A2- 0 699 435
- WO-A-94/18934
- WO-A-94/27571
- WO-A-94/27575
- WO-A-96/32919
- WO-A-98/19656
- US-A- 5 747 435
- 2. AUFLAGE: 'Grundlagen und Rezepturen der Kosmetika', 1989, HOTHIG VERLAG, HEIDELBERG article SCHRADER, KARLHEINZ, pages 677 - 709
- 'International Cosmetic Ingredient Dictionary and Handbook', vol. 10, 2004, CTFA, WASHINGTON pages 287 - 288
- 'International Cosmetic Ingredient Dictionary and Handbook', vol. 7, 1997, CTFA, WASHINGTON pages 1086 - 1091

## Description

La présente invention concerne une composition cosmétique conditionnante et détergente pour le soin et le lavage simultanés des matières kératiniques. L'invention concerne aussi l'utilisation de ladite composition dans l'application susmentionnée.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenues dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Toutefois, et malgré les progrès réalisés récemment dans le domaine des shampooings à base de polymères cationiques en particulier les dérivés cationiques de cellulose ou de gomme de guar et de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

On a déjà utilisé des polymères fortement cationiques dans des compositions pour le lavage ou le soin des cheveux pour faciliter le démêlage des cheveux et pour leur communiquer douceur et souplesse par exemple dans la demande de brevet GB-A-1513672. Cependant, l'usage de ces polymères cationiques dans ce but présente divers inconvénients. En raison de leur forte affinité pour les cheveux, ces polymères se déposent de façon importante lors d'utilisations répétées, et conduisent à des effets indésirables tel qu'un toucher désagréable, un raidissement des cheveux, et une adhésion interfibre affectant le coiffage. Ces inconvénients sont accentués dans le cas de cheveux fins, qui manquent de tenue, de nervosité et de volume.

La présente invention vise à remédier aux inconvénients cités ci-dessus en proposant des compositions conditionnantes et détergentes, suffisamment moussantes, qui présentent de bonnes propriétés de conditionnement, et notamment de démêlage, de douceur et de brillance sans conférer de caractère gras, ni d'effet de charge ou de toucher désagréable.

Ainsi, après de nombreuses recherches menées sur la question, la Demanderesse a maintenant découvert, de façon totalement inattendue et surprenante, qu'en associant une silicone ne comprenant pas de fonction amide et un polymère cationique particulier avec un tensioactif anionique de type carboxylique, il est possible d'obtenir des compositions détergentes présentant d'excellentes propriétés cosmétiques, en particulier de démêlage, de douceur et de brillance et de volume des matières kératiniques traitées et ceci tout en conservant leur bon pouvoir lavant intrinsèque et leur pouvoir moussant.

Ces nouvelles compositions permettent de déposer une quantité importante de silicone sur les matières kératiniques (notamment les cheveux), mais sans toucher ou aspect visuel gras.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse sans aucun effet de charge.

Ces effets sont tout à fait inattendus. En effet, la demanderesse a constaté que l'addition des polymères cationiques décrits ci-après dans des compositions de shampooing à base de tensioactif sulfate et de silicone n'améliorait pas les propriétés cosmétiques de la composition et qu'au contraire des effets de charge apparaissaient.

La présente invention a ainsi pour objet une nouvelle composition cosmétique conditionnante et détergente, caractérisée par le fait qu'elle comprend, dans un milieu aqueux :
(A) au moins un agent tensioactif anionique de type carboxylique ne comprenant pas de fonction sulfate ou sulfonate,
(B) au moins une silicone ne comprenant pas de fonction amide,
(C) au moins un polymère cationique contenant des groupements ammonium quaternaires dans la chaîne principale, et choisi parmi
   (1) les polymères de diammonium quaternaire contenant des motifs récurants répondant à la formule : 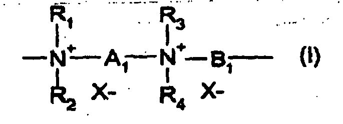 formule (I) dans laquelle :
      R₁, R₂, R₃ et R₄, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁, R₂, R₃ et R₄, ensemble ou séparément, constituent avec les atomes d'azote auxquels ils sont rattachés des hétérocycles contenant éventuellement un second hétéroatome autre que l'azote ou bien R₁, R₂, R₃ et R₄, représentent un radical alkyle en C1-C6 linéaire ou ramifié substitué par un groupement nitrile, ester, acyle, amide ou -CO-O-R₅-D ou -CO-NH-R₅-D où R₅ est un alkylène et D un groupement ammonium quaternaire ;
      A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne polymérique principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, de
      X⁻ désigne un anion dérivé d'un acide minéral ou organique;
      A₁, R₁ et R₃ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)n-CO-D-OC-(CH₂)n- dans lequel D désigne :
         a) un reste de glycol de formule : -O-Z-O-, où Z désigne un radical hydrocarboné linéaire ou ramifié ou un groupement répondant à l'une des formules suivantes :

            -(CH₂CH₂O)ₓ-CH₂CH₂-

            -[CH₂-CH(CH₃)-O]_{y}-CH₂-CH(CH₃)-

            où x et y désignent un nombre entier de 1 à 4, représentant un degré de polymérisation défini et unique ou un nombre quelconque de 1 à 4 représentant un degré de polymérisation moyen ;
         b) un reste de diamine bis-secondaire tel qu'un dérivé de pipérazine ;
         c) un reste de diamine bis-primaire de formule : -NH-Y-NH-, où Y désigne un radical hydrocarboné linéaire ou ramifié, ou bien le radical bivalent -CH₂-CH₂-S-S-CH₂-CH₂- ;
         d) un groupement uréylène de formule : -NH-CO-NH- ;
   (2) les polymères de polyammonium quaternaires constitués de motifs de formule (II): 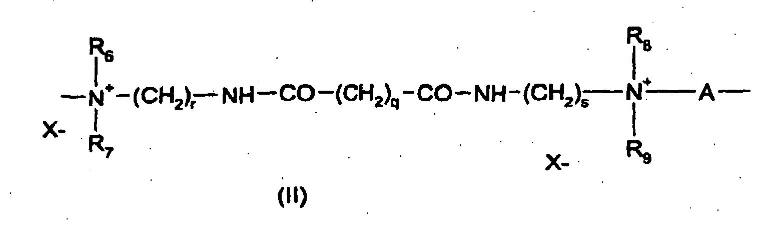 formule dans laquelle :
      R₆, R₇, R₈ et R₉, identiques ou différents, représentent un atome d'hydrogène ou un radica méthyle, éthyle, propyle, β-hydroxyéthyle, β-hydroxypropyle ou -CH₂-CH₂(OCH₂CH₂)ₚOH,
      où p est égal à 0 ou à un nombre entier compris entre 1 et 6, sous réserve que R₆, R₇, R₈ et R₉ ne représentent pas simultanément un atome d'hydrogène,
      r et s, identiques ou différents, sont des nombres entiers compris entre 1 et 6,
      q est égal à 0 ou à un nombre entier compris entre 1 et 34,
      X désigne un atome d'halogène,
      A désigne un radical d'un dihalogénure ou représente de préférence -CH₂-CH₂-O-CH₂-CH₂-.
   (3) Les polymères quaternaires de vinylpyrrolidone et de vinylimidazolium.

La présente invention a également pour objet l'utilisation de la composition selon l'invention pour le soin et le lavage simultanés des matières kératiniques telles que les cheveux et la peau.

Une description détaillée de la présente invention va maintenant être donnée.

Les tensioactifs anioniques de type carboxyliques ne comprenant pas de fonction sulfate ou sulfonate peuvent être notamment choisis parmi les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates et les alkylpolyglycoside tartrate. De tels produits sont notamment vendus sous les dénominations de EUCAROL APG/EC et EUCAROL APG/ET par la société LAMBERTI.
On peut également utiliser les mélanges de ces tensioactifs.

On utilise de préférence les acides alkyl (C₆-C₂₄) éther carboxyliques polyoxyalkylénés, les acides alkyl(C₆-C₂₄) amido éther carboxyliques polyoxyalkylénés en particulier ceux comportant de 2 à 15 groupements oxyde d'alkylène et les esters d'alkyl(C₆-C₂₄)polyglycosides carboxyliques et leurs mélanges.

Les silicones utilisables conformément à l'invention peuvent être solubles ou insolubles dans l'eau ou dans la composition finale. Elles peuvent être volatiles ou non volatiles.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE® 70045 V 2" par RHONE POULENC, le décaméthylcyclopentasiloxane commercialisé sous le nom de "VOLATILE SILICONE 7158" par UNION CARBIDE, "SILBIONE® 70045 V 5" par RHONE POULENC, ainsi que leurs mélanges.
   On peut également citer les cyclocopolymères du type diméthylsiloxanes/méthylakylsiloxane, tel que la "SILICONE VOLATILE FZ 3109" commercialisée par la société UNION CARBIDE, de structure chimique :
   avec D : 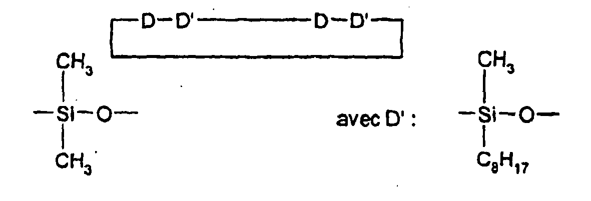 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement (i) les polyalkylsiloxanes ;
(ii) les polyarylsiloxanes ;
(iii) les polyalkylarylsiloxanes ;
(iv) les gommes de silicone ;
(v) les résines de silicone ;
(vi) les polyorganosiloxanes, comportant dans leur structure générale, un ou plusieurs groupements organofonctionnels directement fixés sur la chaîne siloxanique ou fixés par l'intermédiaire d'un radical hydrocarboné ;
(vii) les copolymères blocs ayant un bloc linéaire polysiloxane-polyoxyalkylène comme unités répétitives ;
(viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
(ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
(x) ou leurs mélanges.

Parmi les polyalkylsiloxanes, on peut citer principalement :
- les polydiméthylsiloxanes linéaires à groupements terminaux triméthylsilyle, comme par exemple, et à titre non limitatif; les huiles SILBIONE® de la série 70047 commercialisées par RHONE POULENC, l'huile SILBIONE® 47 V 500 000 de RHONE POULENC ou certaines VISCASIL de la GENERAL ELECTRIC (Viscosil 60.000), les FLUID DC 200 de la société DOW CORNING ou l'huile de silicone AK 300.000 de la société WACKER ;
- les polydiméthylsiloxanes linéaires à groupements terminaux hydroxydiméthylsilyle telles que les huiles de la série 48 V de RHONE POULENC.

Dans cette classe de polyalkylsiloxanes, on peut également mentionner les polyalkylsiloxanes vendus par la société GOLDSCHMIDT sous les dénominations commerciales ABILWAX® 9800 et ABILWAX® 9801 qui sont des polyalkyl(C₁-C₂₀)siloxanes.

Parmi les polyalkylarylsiloxanes, on peut citer les polydiméthylméthylphénylsiloxanes ou les polydiméthyldiphénylsiloxanes, linéaires ou ramifiés tels que le produit DC 556 COSMETIC GRAD FLUID de DOW CORNING.

Les gommes de silicone, conformes à l'invention, sont des polyorganosiloxanes de masse moléculaire moyenne en nombre comprise entre 200.000 et 1.000.000, utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylénes, le chlorure de méthylène, le pentane, le dodécane, le tridécanes ou leurs mélanges.

On cite, par exemple, les composés suivants :
- polydiméthylsiloxane,
- poly[(diméthylsiloxane)/(méthylvinylsiloxane)],
- poly[(diméthylsiloxane)/(diphényisiloxane)],
- poly[(diméthylsiloxane)/(phénylméthylsiloxane)],
- poly[(diméthylsiloxane)/(diphénylsiloxane)/(méthylvinylsiloxane)],

On peut citer, par exemple, les mélanges suivants :
**1**) les mélanges formés à partir d'un polydiméthylsiloxane hydroxylé en bout de chaîne (DIMETHICONOL selon la nomenclature CTFA) et d'un polydiméthylsiloxane cyclique (CYCLOMETHICONE selon la nomenclature CTFA) tels que le produit Q2 1401 vendu par la société DOW CORNING ;
**2**) les mélanges formés à partir d'une gomme de polydiméthylsiloxane avec une silicone cyclique tels que le produit SF 1214 SILICONE FLUID de GENERAL ELECTRIC qui est une gomme SE 30 de poids moléculaire 500.000 solubilisée dans la SF 1202 SILICONE FLUID (décaméthylcyclopentasiloxane) ;
**3**) les mélanges de deux polydiméthylsiloxanes (PDMS) de viscosité différente, notamment d'une gomme PDMS et d'une huile PDMS tels que les produits SF 1236 et CF 1241 de la GENERAL ELECTRIC. Le produit SF 1236 est le mélange d'une huile SE 30 définie ci-dessus de viscosité 20 m²/s et d'une huile SF 96 de viscosité 5.10⁻⁵ m²/s (15% de gomme SE 30 et 85% d'huile SF 96). Le produit CF 1241 est le mélange d'une gomme SE 30 (33%) et d'une PDMS (67%) de viscosité 10⁻³ m²/s.

Les résines de silicone conformes à l'invention sont de préférence des systèmes siloxaniques réticulés renfermant les unités :

R₂SiO_{2/2}, RSiO_{3/2}, SiO_{4/2}, dans lesquelles R désigne un groupe hydrocarboné possédant 1 à 6 atomes de carbone ou un groupement phényle.Parmi ces produits, ceux particulièrement préfères sont ceux où R désigne un radical alkyle inférieur ou phényle.

Parmi ces résines, on peut citer le produit vendu sous le nom DOW CORNING 593 par DOW CORNING ou ceux vendus sous le nom SILICONE FLUID SS 4267 par la GENERAL ELECTRIC et qui sont des diméthyl/triméthypolylsiloxanes.

Les polyorganosiloxanes organomodifiés de l'invention sont des polysiloxanes tels que définis précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les polysiloxanes comportant :
a) des groupements polyéthylèneoxy et/ou polypropylèneoxy, comportant éventuellement des groupes alkyle, tels que le produit dénommé lauryl méthicone copolyol vendu sous le nom Q2 5200 par DOW CORNING ;
b) des groupements (per)fluorés, comme les groupements trifluoroalkyls tels que, par exemple, ceux vendus par SHIN ETSU sous le nom FL 100 ;
c) des groupements thiols ;
d) des groupements carboxylates, tels que les produits décrits dans le brevet européen EP 185 507 de CHISSO CORPORATION ;
e) des groupements hydroxylés, tels que les polyorganopolysiloxanes à fonction hydroxyalkyle décrits dans la demande de brevet français FR 85-16334 et en particulier les polyorganopolysiloxanes à fonction γ- hydroxypropyle ;
f) des groupements alcoxylés comportant au moins 12 atomes de carbone, tels que le produit SILICONE COPOLYMER F755 de SWS SILICONES et les produits ABILWAX® 2428, ABILWAX® 2434, ABILWAX® 2440 de la société GOLDSCHMIDT.
g) des groupements acyloxyalkyls comportant au moins 12 atomes de carbone, tels que les polyorganosiloxanes décrits dans la demande de brevet français FR 88-17433 et en particulier les polyorganosiloxanes à fonction stéaroyloxypropyle.
h) des groupements amphotères;
i) des groupements bisulfites.
j) des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ; On utilise plus particulièrement les silicones dénommées amodiméthicone et triméthylsilylamodiméthicone selon la dénomination CTFA (7^{ème} ed. 1997) ;

Les copolymères blocs ayant un bloc linéaire polysiloxane-polyoxyalkylène comme unités répétitives utilisés dans le cadre de la présente invention ont de préférence la formule générale suivante :

([Y (R₂SiO)ₐ R'₂SiYO] [(CnH₂ₙO)_{b}])_{c} (V)

dans laquelle :
- R et R', identiques ou différents, représentent un radical hydrocarboné monovalent ne contenant pas d'insaturation aliphatique,
- n est un nombre entier allant de 2 à 4,
- a est un nombre entier supérieur ou égal à 5, de préférence compris entre 5 et 200 et encore plus particulièrement entre 5 et 100.
- b est un nombre entier supérieur ou égal à 4, de préférence compris entre 4 et 200 et encore plus particulièrement entre 5 et 100.
- c est un nombre entier supérieur ou égal à 4, de préférence compris entre 4 et 1000 et encore plus particulièrement entre 5 et 300.

- Y représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10 % environ à 95 % environ en poids du copolymère bloc,
- le poids moléculaire moyen en poids du copolymère bloc étant d'au moins 3.000 et de préférence compris entre 5000 et 1000000 et encore plus particulièrement entre 10000 et 200000.

R et R' sont préférentiellement choisis parmi le groupe comprenant les radicaux alkyls comme par exemple les radicaux méthyle, éthyle, propyle, butyle, pentyle, hexyle, octyle, décyle, dodécyle, les radicaux aryls comme par exemple phényle, naphtyle, les radicaux aralkyls comme par exemple benzyle, phényléthyle, les radicaux tolyle, xylyle et cyclohexyle.

Y est de préférence -R''-, -R''-CO-, où R" est un groupe alkylène divalent comme par exemple l'éthylène, le propylène ou le butylène ou un groupe arylène divalent comme - C₆H₄-, -C₆H₄-C₆H₄-, -C₆H₄-CH₂-C₆H₄-, -C₆H₄-C(CH₃-)₂-C₆H₄-.

Encore plus préférentiellement, Y représente un radical alkylène divalent, plus particulièrement le radical -CH₂-CH₂-CH₂- ou le radical C₄H₈.

La préparation des copolymères blocs mis en oeuvre dans le cadre de la présente invention est décrite dans la demande européenne EP 0 492 657 A1, dont l'enseignement est inclus à titre de référence dans la présente description.

Les polymères à squelette organique non-siliconé greffé par des monomères contenant un polysiloxane, conformes à l'invention, sont choisis plus préférentiellement parmi ceux décrits dans les brevets US 4,693,935, US 4,728,571 et US 4,972,037 et les demandes de brevet EP-A-0 412 704, EP-A-0 412 707, EP-A-0 640 105 et WO 95/00578 dont les enseignements sont totalement inclus dans la présente description à titre de références non limitatives. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Des exemples de polymères à squelette polysiloxanique greffé par des monomères organiques non siliconés convenant à la mise en oeuvre de la présente invention, ainsi que leur mode particulier de préparation, sont notamment décrits dans les demandes de brevets EP-A- 0 582 152, WO 93/23009 et WO 95/03776 dont les enseignements sont totalement inclus dans la présente description à titre de références non limitatives.

Selon l'invention, toutes les silicones peuvent également être utilisées sous forme d'émulsions ou de microémulsions.

Les silicones particulièrement préférées conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 Cst, des séries SILBIONE® 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE® 70641 V 200 commercialisée par la société RHONE POULENC ;
- les mélanges d'organopolysiloxanes et de silicones cycliques tels que le produit Q2 1401 commercialisé par la société DOW CORNING, et le produit SF 1214 commercialisé par la société GENERAL ELECTRIC ;
- les mélanges de deux PDMS de viscosités différentes notamment d'une gomme et d'une huile tels que le produit SF 1236 commercialisé par la société GENERAL ELECTRIC ;
- la résine d'organopolysiloxane commercialisée sous la dénomination DOW CORNING 593 ;
- les polysiloxane à groupements aminés tels que les amodiméthicone ou les triméthylsilylamodiméthicone ;

Selon l'invention, la ou les silicone peuvent représenter de 0,05 % à 10 % en poids, de préférence de 0,1 % à 5 % en poids, et encore plus préférentiellement de 0,5 % à 3 % en poids, du poids total de la composition finale.

Les polymères cationiques contenant des groupements ammonium quaternaires dans la chaîne polymérique principale sont choisis parmi :
- (1) les polymères de diammonium quaternaire contenant des motifs récurants répondant à la formule : 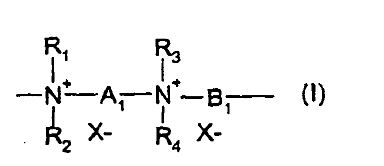 formule (I) dans laquelle :
   R₁, R₂, R₃ et R₄, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁, R₂, R₃ et R₄ ensemble ou séparément, constituent avec les atomes d'azote auxquels ils sont rattachés des hétérocycles contenant éventuellement un second hétéroatome autre que l'azote ou bien R₁, R₂, R₃ et R₄, représentent un radical alkyle en C1-C6 linéaire ou ramifié substitué par un groupement nitrile, ester, acyle, amide ou -CO-O-R₅-D ou -CO-NH-R₅-D où R₅ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁ et R₃ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)n-CO-D-OC-(CH₂)n-
      dans lequel D désigne :
      a) un reste de glycol de formule: -O-Z-O-, où Z désigne un radical hydrocarboné linéaire ou ramifié ou un groupement répondant à l'une des formules suivantes :

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent -CH₂-CH₂-S-S-CH₂-CH₂- ;
      d) un groupement uréylène de formule : -NH-CO-NH- ;

De préférence, X⁻ est un anion tel que le chlorure ou le bromure.

Ces polymères ont une masse moléculaire moyenne en nombre généralement comprise entre 1000 et 1000000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser particulièrement les polymères qui sont constitués de motifs récurrents répondant à la formule : 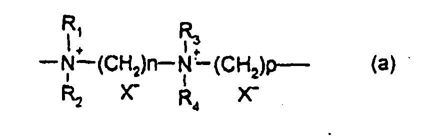 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(2) les polymères de polyammonium quaternaires constitués de motifs de formule (II): 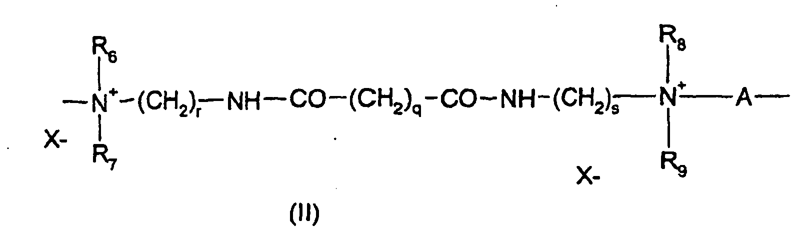 formule dans laquelle :
   R₆, R₇, R₈ et R₉, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
   où p est égal à 0 ou à un nombre entier compris entre 1 et 6, sous réserve que R₆, R₇, R₈ et R₉ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "MIRAPOL A 15", "MIRAPOL AD1", "MIRAPOL AZ1" et "MIRAPOL 175" vendus par la société MIRANOL.
(3) Les polymères quaternaires de vinylpyrrolidone et de vinylimidazolium tels que par exemple les produits commercialisés sous les dénominations Luviquat® FC 905, FC 550 et FC 370 par la société B.A.S.F.

Selon l'invention, le ou les polymères cationiques peuvent représenter de 0,005 % à 10 % en poids, de préférence de 0,01 % à 5 % en poids, et encore plus préférentiellement de 0,1 % à 3 % en poids, du poids total de la composition finale.

Les compositions de l'invention peuvent contenir en outre avantageusement au moins un autre agent tensioactif choisis parmi les agents tensioactifs anioniques de type phosphate, sulfonate et/ou sulfate, les tensioactifs amphotères, les tensioactifs non-ioniques, les tensioactifs cationiques ou leurs mélanges.

Les tensioactifs additionnels convenant à la mise en oeuvre de la présente invention sont notamment les suivants :

### (i) Tensioactif(s) anionique(s).

A titre d'exemple, on peut citer les sels (en particulier sels alcalins, notamment de sodium sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates, les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates; les alkylsulfonates, alkylphosphates, alkylamidesulfonates, alkylarylsulfonates, α-oléfine-sulfonates, paraffine-sulfonates les alkylétherphosphates; les alkyl sulfosuccinamates, les acyl iséthionates, les acyltaurates, le radical alkyle de tous ces différents composés comportant de préférence de 8 à 24 atomes de carbone, et le radical aryl désignant de préférence un groupement phényle ou benzyle, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères additionnels peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle: R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle:
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHONE POULENC.

Parmi les tensioactifs anioniques, on préfère utiliser les sels d'alkyléthersulfates en C₈-C₁₄ et plus particulièrement ceux en C₁₂-C₁₄. Ces sels comprennent notamment de 2 à 5 groupements d'oxyde d'éthylène. On utilise de préférence un agent tensioactif anionique choisi parmi les alkyl (C₁₂-C₁₄)éthersulfates de sodium, de triéthanolamine ou d'ammonium oxyéthylénés à environ 2,2 moles d'oxyde d'éthylène.

Selon la présenté invention, on préfère plus particulièrement utiliser les agents tensioactifs amphotères appartenant au groupe des bétaines tels que les alkylbétaïnes en particulier la cocoylbétaïne commercialisée sous la dénomination « DEHYTON AB 30 » en solution aqueuse à 30 % de MA par la société HENKEL ou les alkylamidobétaines telles que la TEGOBETAINE® F50 commercialisée par la société GLODSCHMIDT.

Le(s) agent(s) tensioactif(s) anionique(s) de type carboxylate sont généralement présents à raison de 3 à 50 % en poids, de préférence de 3 à 20 % en poids, par rapport au poids total de la composition.

Le(s) agent(s) tensioactif(s) anionique(s) ne contenant pas de groupement carboxylate sont généralement présents à raison de 1 à 30 % en poids, de préférence de 3 à 15 % en poids, par rapport au poids total de la composition.

Le(s) agent(s) tensioactif(s) amphotère(s) ou non ioniques sont généralement présents à raison de 0,5 à environ 15% en poids, de préférence de 1 à 5% en poids, par rapport au poids total de la composition.

Le rapport en poids tensioactifs de type carboxylate/totalité des tensioactifs peut varier de 0,1 à 1 et de préférence de 0,2 à 1.

Dans la composition selon la présente invention, la totalité des tensioactifs détergents représente généralement de 3 à 50% en poids et de préférence de 5 à 30% en poids par rapport au poids total de la composition.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 5 et 8. L'ajustement du pH à la valeur désirée peut se faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination « AMINOL A15 » par la société CHEM Y, les acides polyacryliques réticulés et les copolymères acide acrylique / acrylates d'alkyle en C₁₀-C₃₀ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les alcools gras, les éthers à chaînes grasses tels que par exemple le distéaryléther ou le 1-(hexadécyloxy)-2-octadécanol.

Les compositions conformes à l'invention peuvent éventuellement contenir en outre d'autres agents ayant pour effet d'améliorer les propriétés cosmétiques de cheveux ou de la peau sans cependant altérer la stabilité et/ou les propriétés lavantes et moussantes des compositions. On peut citer à ce sujet les agents tensioactifs cationiques, les polymères anioniques, les polymères non ioniques, les polymères cationiques différents de ceux de l'invention, les polymères amphotères, les protéines, les hydrolysat de protéine, les acides aminés, les céramides, les pseudocéramides, les acides gras à chaînes linéaires ou ramifiées en C₁₆-C₄₀ tels que l'acide méthyl-18 eicosanoique, les hydroxyacides, les vitamines, le panthénol, les esters d'acides gras, les silicones différentes de celles de l'invention, les agents hydratants, les agents antipelliculaires ou antiséborrhéiques, les filtres solaires, les agents anti-radicaux libres, les huiles minérales, les huiles végétales, les huiles organiques de synthèse et leurs mélanges.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Ces compositions peuvent se présenter sous la forme de liquides plus ou moins épaissis, de crèmes ou de gels et elles conviennent principalement au lavage et au soin des cheveux.

Lorsque les compositions conformes à l'invention sont mises en oeuvre comme des shampooings, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions conformes à l'invention sont également utilisables comme gels douche, bains moussants, comme produits démaquillants moussants, pour le lavage et le conditionnement des cheveux et/ou de la peau, auquel cas ils sont appliqués sur la peau et/ou les cheveux humides et sont rincés après application.

Des exemples concrets, mais nullement limitatifs, illustrant l'invention vont maintenant être donnés.

### EXEMPLE 1 :

On a réalisé deux compositions de shampooings, l'une conforme à l'invention (composition A) et l'autre comparative (composition B) :(MA signifie Matière Active) :

| | A | B |
|---|---|---|
| - Lauryl amido ether(3 OE) carboxylate de sodium en solution à 30% de MA dans l'eau (AKYPO FOAM 30 BV de KAO) | 14 g MA | - |
| - Alkyl (C12-C14)éther sulfate de sodium oxyéthyléné à 2,2 moles d'oxyde d'éthylène en solution aqueuse à 70% de MA | - | 14 gMA |
| - Hexadimethrine chloride en solution aqueuse à 60% MA (MEXOMER PC de CHIMEX) | 0,6 g MA | 0,6 g MA |
| - Polydiméthylsiloxane en émulsion non ionique aqueuse à 50% de MA (DC2-1691 de DOW CORNING) | 2,5 g MA | 2,5 g MA |
| - Gomme de xanthane (KELTROL T de NUTRASWEET KELCO) | 1 g | 1 g |
| - Conservateurs | qs | qs |
| | pH 7 | pH 7 |
| - Eau déminéralisée qsp | 100 g | 100 g |

On effectue un shampooing en appliquant environ 1 g de la composition A sur des mèches de 2,5 g de cheveux naturels préalablement mouillés. On fait mousser le shampooing, on laisse pauser pendant 10 minutes puis on rince abondamment à l'eau.

On procède selon le même mode opératoire que ci-dessus avec la composition comparative B.
Un panel d'experts a évalué l'aspect des cheveux mouillés.

Tous les experts indiquent que les cheveux traités avec la composition A selon l'invention sont plus doux et se démêlent facilement que ceux traités avec la composition B. Les cheveux traités avec la composition A ont un toucher non chargé.

### EXEMPLE 2 :

On a réalisé deux compositions de shampooings, l'une conforme à l'invention (composition A) et l'autre comparative (composition B) :(MA signifie Matière Active) :

| | A | B |
|---|---|---|
| - Lauryl amido ether(3 OE) carboxylate de sodium en solution à 30% de MA dans l'eau (AKYPO FOAM 30 BV de KAO) | 10 g MA | - |
| - Alkyl (C12-C14)éther sulfate de sodium oxyéthyléné à 2,2 moles d'oxyde d'éthylène en solution aqueuse à 70% de MA | 4 gMA | 14 gMA |
| - Hexadimethrine chloride en solution aqueuse à 60% MA (MEXOMER PO de CHIMEX) | 0,6 g MA | 0,6 g MA |
| - Polydiméthylsiloxane en émulsion non ionique aqueuse à 50% de MA (DC2-1691 de DOW CORNING) | 2,5 g MA | 2,5 g MA |
| - Gomme de xanthane (KELTROL T de NUTRASWEET KELCO) | 1 g | 1 g |
| - Conservateurs | qs | qs |
| | pH 7 | pH 7 |
| - Eau déminéralisée qsp | 100 g | 100 g |

On effectue un shampooing en appliquant environ 1 g de la composition A sur des mèches de 2,5 g de cheveux naturels préalablement mouillés. On fait mousser le shampooing, on laisse pauser pendant 10 minutes puis on rince abondamment à l'eau.

On procède selon le même mode opératoire que ci-dessus avec la composition comparative B.
Un panel d'experts a évalué l'aspect des cheveux mouillés.

Tous les experts indiquent que les cheveux traités avec la composition A selon l'invention sont plus doux et se démêlent facilement que ceux traités avec la composition B. Les cheveux traités avec la composition A ont un toucher non chargé.

### EXEMPLE 3 :

On a réalisé deux compositions de shampooings, l'une conforme à l'invention (composition A) et l'autre comparative (composition B) :(MA signifie Matière Active) :

| | A (Invention) | B (Ex. 10 de US5180584) |
|---|---|---|
| - Acide nonylphénol éther carboxylique oxyéthyléné (7 OE) (AKYPO NP 70 de CHEM Y) | 7,2 g MA | 7,2 g MA |
| - Alkyl (C12-C14) sulfate de triéthanolamine en solution aqueuse à 40% de MA | 10 gMA | 10 gMA |
| - Laurylsarcosinate de sodium en solution aqueuse à 30% de MA (ORAMIX L30 de SEPPIC) | 4 gMA | 4 gMA |
| - Hexadimethrine chloride en solution aqueuse à 60% MA (MEXOMER PO de CHIMEX) | 1 g MA | - |
| - Huile de silicone (Huile 70633 V 30 de RHONE POULENC) | 2 g | 2 g |
| - Diméthicone copolyol (Huile CL 183/25 de GOLDSCHMIDT) | 5 g MA | 5 g MA |
| - Hydroxyéthylcellulose réticulée par de l'épichlorhydrine et quaternisée par de la triéthanolamine (JR 400 de UNION CARBIDE) | - | 1 gMA |
| - Triéthanolamine qs | pH 6 | pH 6 |
| - Eau déminéralisée qsp | 100 g | 100 g |

On effectue un shampooing en appliquant environ g de la composition A sur des mèches de 2,5 g de cheveux naturels préalablement mouillés. On fait mousser le shampooing, on laisse pauser pendant 10 minutes puis on rince abondamment à l'eau. On procède selon le même mode opératoire que ci-dessus avec la composition comparative B.
Un panel d'experts a évalué l'aspect des cheveux mouillés.

90% des experts indiquent que les cheveux traités avec la composition A selon l'invention sont plus doux et plus lisses et se démêlent facilement que ceux traités avec la composition B.
Les cheveux traités avec la composition A ont un toucher non chargé.

### EXEMPLE 4 :

On a réalisé deux compositions de shampooings, l'une conforme à l'invention (composition A) et l'autre comparative (composition B) :(MA signifie Matière Active) :

| | A (Invention) | B (Ex. 17 de US5180584) |
|---|---|---|
| - Acide nonylphénol éther carboxylique oxyéthyléné (7 OE) (AKYPO NP 70 de CHEM Y) | 4,5 g MA | 4,5 g MA |
| - Alkyl (C12-C14) sulfate de sodium en solution aqueuse à 40% de MA | 10 gMA | 10 gMA |
| - Laurylbétaïne en solution aqueuse à 30% de MA (DEHYTON AB 30 de HENKEL) | 5 gMA | 5 gMA |
| - Hexadimethrine chloride en solution aqueuse à 60% MA (MEXOMER PO de CHIMEX) | 0,5 g MA | - |
| - Protéine quaternisée en solution aqueuse à 30% de MA (LEXEIN QX 3000 de INOLEX) | - | 0,5 gMA |
| - Résine de silicone (DC 593 de DOW CORNING) | 0,5 g | 0,5 g |
| - Chlorure de stéaryl diméthyl benzyl ammonium | 1 g | 1 g |
| pH spontané | 3,6 | 3,6 |
| -Eau déminéralisée qsp | 100 g | 100 g |

On effectue un shampooing en appliquant environ 1 g de la composition A sur des mèches de 2,5 g de cheveux naturels préalablement mouillés. On fait mousser le shampooing, on laisse pauser pendant 10 minutes puis on rince abondamment à l'eau. On procède selon le même mode opératoire que ci-dessus avec la composition comparative B.

Un panel d'experts a évalué l'aspect des cheveux mouillés.

90% des experts indiquent que les cheveux traités avec la composition A selon l'invention sont plus doux et plus lisses et se démêlent facilement que ceux traités avec la composition B.
Les cheveux traités avec la composition A ont un toucher non chargé.

## Revendications

1. Composition cosmétique conditionnante et détergente, **caractérisée par le fait qu'**elle comprend, dans un milieu aqueux :
(A) au moins un agent tensioactif anionique de type carboxylique ne comprenant pas de fonction sulfate ou sulfonate,
(B) au moins une silicone ne comprenant pas de fonction amide,
(C) au moins un polymère cationique contenant des groupements ammonium quaternaires dans la chaîne polymérique principale,
ledit polymère cationique est choisi parmi :
- (1) les polymères de diammonium quaternaire contenant des motifs récurants répondant à la formule : 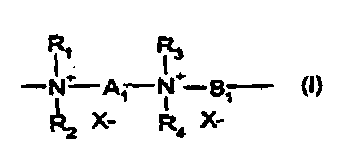 formule (I) dans laquelle :
R₁, R₂, R₃ et R₄, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁, R₂, R₃ et R₄, ensemble ou séparément, constituent avec les atomes d'azote auxquels ils sont rattachés des hétérocycles contenant éventuellement un second hétéroatome autre que l'azote ou bien R₁, R₂, R₃ et R₄, représentent un radical alkyle en C1-C6 linéaire ou ramifié substitué par un groupement nitrile, ester, acyle, amide ou -CO-O-R₅-D ou - CO-NH-R₅-D où R₅ est un alkylène et D un groupement ammonium quaternaire ;
A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne polymérique principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A₁, R₁ et R₃ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)n-CO-D-OC-(CH₂)n-
dans lequel D désigne :
a) un reste de glycol de formule : -O-Z-O-, où Z désigne un radical hydrocarboné linéaire ou ramifié ou un groupement répondant à l'une des formules suivantes :
-(CH₂CH₂O)ₓ-CH₂CH₂-
-[CH₂-CH(CH₃)-O]_{y}-CH₂-CH(CH₃)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent -CH₂-CH₂-S-S-CH₂-CH₂- ;
d) un groupement uréylène de formule : -NH-CO-NH- ;
(2) les polymères de polyammonium quaternaires constitués de motifs de formule (II): 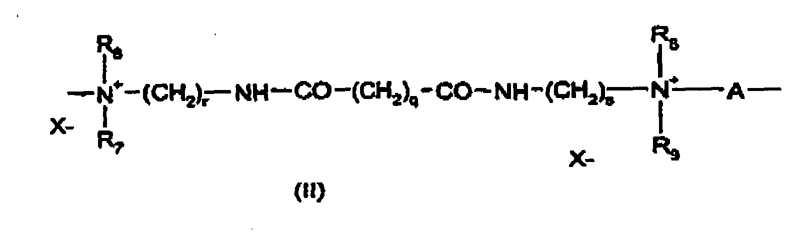 formule dans laquelle :
R₆, R₇, R₈ et R₉, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
où p est égal à 0 ou à un nombre entier compris entre 1 et 6, sous réserve que R₆, R₇, R₈ et R₉ ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH₂-CH₂-O-CH₂-CH₂-.
(3) Les polymères quaternaires de vinylpyrrolidone et de vinylimidazolium.

2. Composition selon la revendication 1, **caractérisée par le fait que** l'agent tensioactif anionique de type carboxylique est choisi parmi les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, les alkyl(C₆-C₂₄) sulfosuccinates, les alkyl(C₆-C₂₄) éthersulfosuccinates, les alkyl(C₆-C₂₄) amidesulfosuccinates; les alkyl(C₆-C₂₄) sulfoacétates ; les acyl(C₆-C₂₄) sarcosinates ; les acyl(C₆-C₂₄)glutamates, les esters d'alkyl(C₆-C₂₄) polyglycosides carboxyliques, et leurs mélanges.

3. Composition selon la revendication 2, **caractérisée par le fait que** ledit agent tensioactif anionique de type carboxylique est choisi parmi les acides alkyl (C₆-C₂₄) éther carboxyliques polyoxyalkylénés, les acides alkyl(C₆-C₂₄) amido éther carboxyliques polyoxyalkylénés en particulier ceux comportant de 2 à 15 groupements oxyde d'alkylène et les esters d'alkyl(C₆-C₂₄)polyglycosides carboxyliques et leurs mélanges.

4. Composition selon l'une quelconque des revendications 1 à 3, **caractérisée par le fait que** les silicones sont choisies dans le groupe constitué par :
(i) les polyalkylsiloxanes ;
(ii) les polyarylsiloxanes ;
(iii) les polyalkylarylsiloxanes ;
(iv) les gommes de silicone ;
(v) les résines de silicone ;
(vi) les polyorganosiloxanes, comportant dans leur structure générale, un ou plusieurs groupements organofonctionnels directement fixés sur la chaîne siloxanique ou fixés par l'intermédiaire d'un radical hydrocarboné ;
(vii) les copolymères blocs ayant un bloc linéaire polysiloxane-polyoxyalkylène comme unités répétitives ;
(viii) les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ;
(ix) les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ;
(x) ou leurs mélanges.

5. Composition selon l'une quelconque des revendications 1 à 4, **caractérisée par le fait que** le polymère cationique est choisi parmi les polymères qui sont constitués de motifs récurrents répondant à la formule : 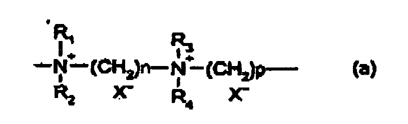 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

6. Composition selon l'une quelconque des revendications 1 à 5, **caractérisée en ce que** la composition comprend en outre au moins un autre agent tensioactif choisis parmi les agents tensioactifs anioniques de type phosphate, sulfonate et/ou sulfate, les tensioactifs amphotères, les tensioactifs non-ioniques, les tensioactifs cationiques ou leurs mélanges.

7. Composition selon l'une quelconque des revendications 1 à 6, **caractérisée par le fait que** la silicone est présente dans des concentrations allant de 0,05 % à 10 % en poids, de préférence de 0,1 % à 5 % en poids, et encore plus préférentiellement de 0,5 % à 3 % en poids, du poids total de la composition finale.

8. Composition selon l'une quelconque des revendications 1 à 7, **caractérisée par le fait que** ledit tensioactif anionique de type carboxylique est présent dans des concentrations comprises entre 3 et 50 % en poids, de préférence de 3 à 20 % en poids, par rapport au poids total de la composition.

9. Composition selon l'une quelconque des revendications 1 à 8, **caractérisée par le fait que** ledit polymère cationique est présent dans des concentrations allant de 0,005 % à 10 % en poids, de préférence de 0,01 % à 5 % en poids, et encore plus préférentiellement de 0,1 % à 3 % en poids, du poids total de la composition finale.

10. Composition selon l'une quelconque des revendications 1 à 9, **caractérisée par le fait que** la totalité des tensioactifs détergents est comprise entre 3 et 50% en poids par rapport au poids total de la composition.

11. Composition cosmétique selon l'une quelconque des revendications 1 à 10, **caractérisée par le fait qu'**elle contient en plus un ou plusieurs adjuvants choisis par les tensioactifs cationiques, les polymères anioniques, les polymères non ioniques, les polymères cationiques, les polymères amphotères, les protéines, les hydrolysat de protéine, les acides aminés, les céramides, les pseudocéramides, les hydroxyacides, les vitamines, le panthénol, les agents hydratants, les agents antipelliculaires ou antiséborrhéiques, les filtres solaires, les agents anti radicaux libres, et leurs mélanges.

12. Utilisation de la composition cosmétique telle que définie à l'une quelconque des revendications 1 à 11 pour le soin et le lavage simultanés des matières kératiniques telles que les cheveux et la peau.

13. Procédé de lavage et de conditionnement des matières kératiniques telles que les cheveux consistant à appliquer sur lesdites matières mouillées une quantité efficace d'une composition telle que définie dans l'une quelconque des revendications 1 à 11 puis à effectuer un rinçage à l'eau après un éventuel temps de pause.

## Claims

1. Cosmetic conditioning and detergent composition, **characterized in that** it comprises in an aqueous medium
(A) at least one anionic surfactant of carboxylic type which does not include a sulphate or sulphonate function,
(B) at least one silicone which does not include an amide function, and
(C) at least one cationic polymer which contains quaternary ammonium groups in the polymeric main chain, the said cationic polymer being selected from:
(1) quaternary diammonium polymers containing repeating units of the formula: 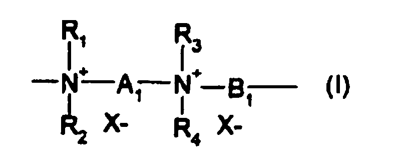 in which formula (I):
R₁, R₂, R₃ and R₄, which are identical or different, represent aliphatic, alicyclic or arylaliphatic radicals containing 1 to 20 carbon atoms or lower hydroxyalkyl aliphatic radicals, or else R₁, R₂, R₃ and R₄, together or separately, together with the nitrogen atoms to which they are attached, form heterocycles optionally containing a second heteroatom other than nitrogen, or else R₁, R₂, R₃ and R₄ represent a linear or branched C₁-C₆ alkyl radical substituted by a nitrile, ester, acyl or amide group or by a group -CO-O-R₅-D or -CO-NH-R₅-D in which R₅ is an alkylene and D is a quaternary ammonium group;
A₁ and B₁ represent polymethylene groups which contain 2 to 20 carbon atoms, can be linear or branched and saturated or unsaturated and can contain, bonded to or intercalated in the polymeric main chain, one or more aromatic rings, or one or more oxygen atoms, sulphur atoms or sulphoxide, sulphone, disulphide, amino, alkylamino, hydroxyl, quaternary ammonium, ureido, amide or ester groups, and
X⁻ denotes an anion derived from a mineral or organic acid;
A₁, R₁ and R₃ can, together with the two nitrogen atoms to which they are attached, form a piperazine ring; and, if A₁ denotes a linear or branched, saturated or unsaturated alkylene or hydroxyalkylene radical, B₁ may also denote a group (CH₂) ₙ-CO-D-OC- (CH₂) ₙ-
in which D denotes:
a) a glycol residue of formula -O-Z-O-, in which Z denotes a linear or branched hydrocarbon radical or a group corresponding to one of the following formulae:
- (CH₂-CH₂-O)ₓ-CH₂-CH₂-
-[CH₂-CH(CH₃ -O]_{y}-CH₂-CH(CH₃)-
in which x and y denote an integer from 1 to 4, representing a defined and single degree of polymerization, or any number from 1 to 4, representing an average degree of polymerization;
b) a bis-secondary diamine residue, such as a piperazine derivative;
c) a bis-primary diamine residue of formula -NH-Y-NH-, in which Y denotes a linear or branched hydrocarbon radical, or else the divalent radical -CH₂-CH₂-S-S-CH₂-CH₂-;
d) a ureylene group of formula -NH-CO-NH-,
(2) quaternary polyammonium polymers consisting of units of formula (II): 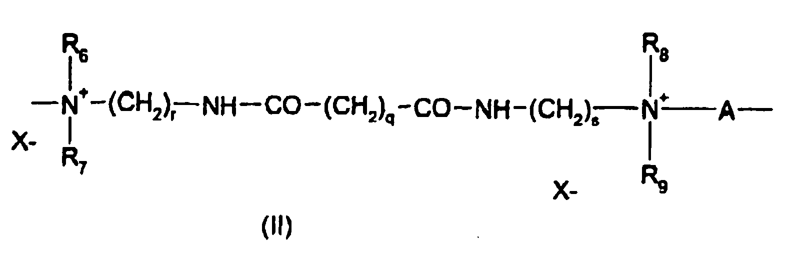 in which formula:
R₆, R₇, R₈ and R₉, which are identical or different, represent a hydrogen atom or a methyl, ethyl, propyl, β-hydroxyethyl, β-hydroxypropyl or -CH₂CH₂(OCH₂CH₂)ₚOH radical,
in which p is 0 or an integer between 1 and 6, with the proviso that R₆, R₇, R₈ and R₉ do not simultaneously represent a hydrogen atom,
r and s, which are identical or different, are integers between 1 and 6,
q is 0 or an integer between 1 and 34,
X denotes a halogen atom,
A denotes a radical of a dihalide or represents, preferably, -CH₂-CH₂-O-CH₂-CH₂-,
(3) quaternary polymers of vinylpyrrolidone and vinylimidazolium.

2. Composition according to Claim 1, **characterized in that** the anionic surfactant of carboxylic type is selected from alkyl-D-galactoside-uronic acids and their salts, polyalkoxylated C₆-C₂₄-alkyl ether carboxylic acids, polyalkoxylated (C₆-C₂₄-alkyl)aryl ether carboxylic acids, polyalkoxylated C₆-C₂₄-alkylamido ether carboxylic acids and their salts, in particular those containing from 2 to 50 alkylene oxide groups, especially ethylene oxide groups, C₆-C₂₄-alkyl sulphosuccinates, C₆-C₂₄-alkyl ether sulphosuccinates, C₆-C₂₄-alkylamide sulphosuccinates; C₆-C₂₄-alkyl sulphoacetates; (C₆-C₂₄-acyl)sarcosinates; (C₆-C₂₄-acyl)glutamates, (C₆-C₂₄-alkyl)polyglycoside carboxylic esters, and mixtures thereof.

3. Composition according to Claim 2, **characterized in that** the said anionic surfactant of carboxylic type is selected from polyalkoxylated C₆-C₂₄-alkyl ether carboxylic acids, polyalkoxylated C₆-C₂₄-alkylamido ether carboxylic acids, especially those containing 2 to 15 alkylene oxide groups, and (C₆-C₂₄-alkyl)polyglycoside carboxylic esters and mixtures thereof.

4. Composition according to any one of Claims 1 to 3, **characterized in that** the silicones are selected from the group consisting of:
(i) polyalkylsiloxanes;
(ii) polyarylsiloxanes;
(iii) polyalkylarylsiloxanes;
(iv) silicone gums;
(v) silicone resins;
(vi) polyorganosiloxanes comprising in their general structure one or more organic functional groups attached directly to the siloxane chain or attached via a hydrocarbon radical;
(vii) block copolymers having a linear polysiloxane-polyoxyalkylene block as repeating units;
(viii) graft silicone polymers, having a non-silicone organic framework, which consist of an organic main chain which is formed from non-silicone organic monomers and onto which there is grafted, within the said chain and optionally at at least one of its ends, at least one polysiloxane macromonomer;
(ix) graft silicone polymers, having a polysiloxane framework grafted with non-silicone organic monomers, which comprise a polysiloxane main chain to which there is grafted, within the said chain and optionally at at least one of its ends, at least one non-silicone organic macromonomer;
(x) or mixtures thereof.

5. Composition according to any one of Claims 1 to 4, **characterized in that** the cationic polymer is selected from polymers which consist of repeating units corresponding to the formula: 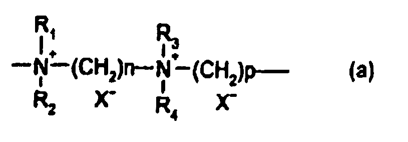 in which R₁, R₂, R₃ and R₄, which are identical or different, denote an alkyl or hydroxyalkyl radical having approximately 1 to 4 carbon atoms, n and p are integers varying from 2 to 20 approximately, and X⁻ is an anion derived from a mineral or organic acid.

6. Composition according to any one of Claims 1 to 5, **characterized in that** the composition further comprises at least one other surfactant, selected from anionic surfactants of phosphate, sulphonate and/or sulphate type, amphoteric surfactants, nonionic surfactants, cationic surfactants and mixtures thereof.

7. Composition according to any one of Claims 1 to 6, **characterized in that** the silicone is present in concentrations ranging from 0.05% to 10% by weight, preferably from 0.1% to 5% by weight and, more preferably still, from 0.5% to 3% by weight of the total weight of the final composition.

8. Composition according to any one of Claims 1 to 7, **characterized in that** the said anionic surfactant of carboxylic type is present in concentrations of between 3 and 50% by weight, preferably from 3 to 20% by weight, relative to the total weight of the composition.

9. Composition according to any one of Claims 1 to 8, **characterized in that** the said cationic polymer is present in concentrations ranging from 0.005% to 10% by weight, preferably from 0.01% to 5% by weight and, more preferably still, from 0.1% to 3% by weight of the total weight of the final composition.

10. Composition according to any one of Claims 1 to 9, **characterized in that** the entirety of the detergent surfactants is between 3 and 50% by weight relative to the total weight of the composition.

11. Cosmetic composition according to any one of Claims 1 to 10, **characterized in that** it additionally comprises one or more adjuvants selected from cationic surfactants, anionic polymers, nonionic polymers, cationic polymers, amphoteric polymers, proteins, protein hydrolysates, amino acids, ceramides, pseudoceramides, hydroxy acids, vitamins, panthenol, moisturizers, antidandruff or antiseborrhoeic agents, sunscreens, free-radical scavengers, and mixtures thereof.

12. Use of the cosmetic composition as defined in any one of Claims 1 to 11 for the simultaneous care and washing of keratinous substances such as the hair and skin.

13. Method of washing and conditioning keratinous substances such as the hair, which consists in applying an effective amount of a composition as defined in any one of Claims 1 to 11 to the said substances, which have been wetted, and then in carrying out rinsing with water after an optional waiting period.

## Patentansprüche

1. Reinigende und konditionierende, kosmetische Zusammensetzung, **dadurch gekennzeichnet, dass** sie in einem wässrigen Medium enthält:
(A) mindestens einen anionischen grenzflächenaktiven Stoff vom Carboxytyp, der keine Sulfat- oder Sulfonatfunktion enthält,
(B) mindestens ein Silicon, das keine Amidfunktion aufweist, und
(C) mindestens ein kationisches Polymer, das in der Polymerhautkette quartäre Ammoniumgruppen enthält, wobei das kationische Polymer ausgewählt ist unter:
(1) den quartären Diammoniumpolymeren mit wiederkehrenden Einheiten der folgenden Formel: 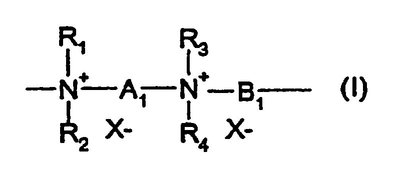 wobei in der Formel (I):
die Gruppen R₁, R₂, R₃ und R₄, die identisch oder voneinander verschieden sind, aliphatische, alicyclische oder arylaliphatische Gruppen mit 1 bis 20 Kohlenstoffatomen oder niedere hydroxyalkylaliphatische Gruppen bedeuten oder wobei die Gruppen R₁, R₂, R₃ und R₄ gemeinsam oder unabhängig voneinander mit den Stickstoffatomen, an die sie gebunden sind, Heterocyclen bilden, die gegebenenfalls ein zweites Heteroatom enthalten, das von Stickstoff verschieden ist, oder wobei die Gruppen R₁, R₂, R₃ und R₄ eine geradkettige oder verzweigte C₁₋₆-Alkylgruppe bedeuten, die mit einer Nitril-, Ester-, Acyl- oder Amidgruppe oder -CO-O-R₅-D oder -CO-NH-R₅-D substituiert ist, worin R₅ eine Alkylengruppe und D eine quartäre Ammoniumgruppe bedeuten;
A₁ und B₁ bedeuten Polymethylengruppen mit 2 bis 20 Kohlenstoffatomen, die geradkettig oder verzweigt, gesättigt oder ungesättigt sein können und die an die Hauptkette gebunden oder in der Hauptkette einen oder mehrere aromatische Ringe oder ein oder mehrere Sauerstoffatome oder Schwefelatome oder eine oder mehrere der folgenden Gruppen enthalten können: Sulfoxid, Sulfon, Disulfid, Amino, Alkylamino, Hydroxy, quartäre Ammoniumgruppen, Ureido, Amid oder Ester;
X- bedeutet ein Anion, das von einer anorganischen oder organischen Säure abgeleitet ist;
Die Gruppen A₁, R₁ und R₃ können mit den beiden Stickstoffatomen, an die sie gebunden sind, einen Piperazinring bilden; wenn A₁ eine geradkettige oder verzweigte, gesättigte oder ungesättigte Alkylengruppe oder Hydroxyalkylengruppe bedeutet, kann die Gruppe B₁ auch eine Gruppe (CH₂)ₙ-CO-D-OC-(CH₂)ₙ- bedeuten, worin D bedeutet:
a) eine Glykolgruppe der Formel : -O-Z-O- , worin Z eine geradkettige oder verzweigte Kohlenwasserstoffgruppe oder eine Gruppe bedeutet, die einer der folgenden Formeln entspricht:
-(CH₂-CH₂-O)ₓ-CH₂-CH₂-
-[CH₂-CH(CH₃)-O]_{y}-CH₂-CH(CH₃)-
worin x und y ganze Zahlen von 1 bis 4 bedeuten und einen wohl definierten und einzigen Polymerisationsgrad darstellen, oder beliebige Zahlen von 1 bis 4 bedeuten und einen mittleren Polymerisationsgrad darstellen;
b) ein bis-sekundäres Diamin, beispielsweise ein Piperazinderivat,
c) ein bis-primäres Diamin der Formel : -NH-Y-NH- , worin Y eine geradkettige oder verzweigte Kohlenwasserstoffgruppe oder auch die zweiwertige Gruppe -CH₂-CH₂-S-S-CH₂-CH₂- bedeutet, oder
d) die Ureylengruppe der Formel : -NH-CO-NH-;
(2) Quartären Polyammoniumpolymeren, die aus Einheiten der folgenden Formel (II) bestehen: 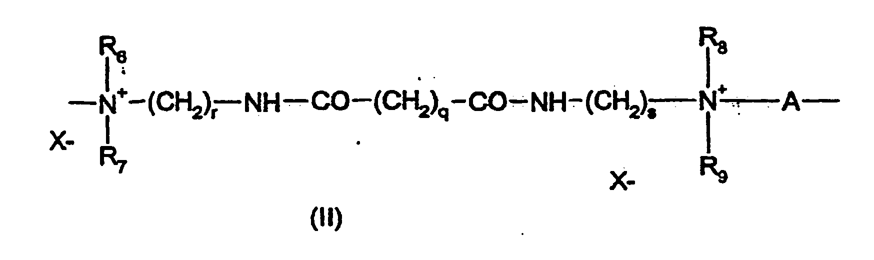 wobei in der Formel bedeuten:
R₆, R₇, R₈ und R₉, die identisch oder voneinander verschieden sind, ein Wasserstoffatom oder Methyl, Ethyl, Propyl, β-Hydroxyethyl, β-Hydroxypropyl oder -CH₂CH₂(OCH₂CH₂)ₚOH, wobei p Null oder eine ganze Zahl im Bereich von 1 bis 6 bedeutet, mit der Maßgabe, dass R₆, R₇, R₈ und R₉ nicht gleichzeitig Wasserstoff bedeuten,
r und s, die gleich oder verschieden sind, eine ganze Zahl im Bereich von 1 bis 6,
q Null oder eine ganze Zahl im Bereich von 1 bis 34,
X ein Halogenatom, und
A ein Dihalogenid oder vorzugsweise -CH₂-CH₂-O-CH₂-CH₂-;
(3) Quartären Polymeren von Vinylpyrrolidon und Vinylimidazol.

2. Zusammensetzung nach Anspruch 1, **dadurch gekennzeichnet, dass** der anionische grenzflächenaktive Stoff vom Carboxytyp unter den Alkyl-D-galactosiduronsäuren und deren Salzen, polyalkoxylierten Alkyl(C₆₋₂₄)ethercarbonsäuren, polyalkoxylierten Alkyl(C₆₋₂₄)arylethercarbonsäuren, polyalkoxylierten Alkyl(C₆₋₂₄)-amidoethercarbonsäuren und ihren Salzen, insbesondere Verbindungen, die 2 bis 50 Alkylenoxidgruppen, besonders Ethylenoxidgruppen, aufweisen, Alkyl(C₆₋₂₄)sulfosuccinaten, Alkyl(C₆₋₂₄)-ethersulfosuccinaten, Alkyl(C₆₋₂₄)amidsulfosuccinaten, Alkyl(C₆₋₂₄) sulfoacetaten, Acyl(C₆₋₂₄)sarcosinaten, Acyl(C₆₋₂₄)glutamaten, Alkyl(C₆₋₂₄)polyglykosidcarbonsäureestern und deren Gemischen ausgewählt ist.

3. Zusammensetzung nach Anspruch 2, **dadurch gekennzeichnet, dass** der anionische grenzflächenaktive Stoff vom Carboxytyp unter den polyalkoxylierten Alkyl(C₆₋₂₄)ethercarbonsäuren, polyalkoxylierten C₆₋₂₄-Alkyl(C₆₋₂₄)amidoethercarbonsäuren, insbesondere Verbindungen, die 2 bis 15 Alkylenoxidgruppen aufweisen, und Alkyl(C₆₋₂₄)polyglykosidcarbonsäureestern und deren Gemischen ausgewählt ist.

4. Zusammensetzung nach einem der Ansprüche 1 bis 3, **dadurch gekennzeichnet, dass** die Silicone ausgewählt sind unter:
(i) Polyalkylsiloxanen;
(ii) Polyarylsiloxanen;
(iii) Polyalkylarylsiloxanen;
(iv) Silicongummis;
(v) Siliconharzen;
(vi) Polyorganosiloxanen, die in ihrer allgemeinen Struktur eine oder mehrere organofunktionelle Gruppen aufweisen, die direkt oder über eine Kohlenwasserstoffgruppe an die Siloxankette gebunden sind;
(vii) Blockcopolymeren, die als wiederkehrende Einheiten einen geradkettigen Polysiloxan-Polyoxyalkylen-Block enthalten;
(viii) gepfropften Siliconpolymeren mit einem nicht siliconhaltigen organischen Grundgerüst, die aus einer organischen Hauptkette bestehen, die aus organischen Monomeren gebildet wird, die kein Silicon enthalten, auf die im Inneren der Kette sowie gegebenenfalls an mindestens einem ihrer Enden mindestens ein Polysiloxanmakromer gepfropft ist;
(ix) gepfropften Siliconpolymeren mit Polysiloxangerüst, auf das nicht siliconhaltige organische Monomere gepfropft sind, die eine Polysiloxanhauptkette enthalten, auf die im Inneren der Kette sowie gegebenenfalls an mindestens einem Kettenende mindestens ein organisches Makromonomer gepfropft ist, das kein Silicon enthält;
(x) oder deren Gemischen.

5. Zusammensetzung nach einem der Ansprüche 1 bis 4, **dadurch gekennzeichnet, dass** das kationische Polymer unter den Polymeren ausgewählt ist, die aus wiederkehrenden Einheiten der folgenden Formel bestehen: 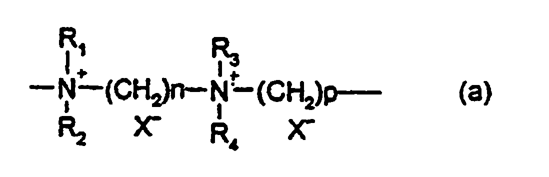 worin die Gruppen R₁, R₂, R₃ und R₄, die gleich oder verschieden sind, eine Alkyl- oder Hydroxyalkylgruppe mit etwa 1 bis 4 Kohlenstoffatomen bedeuten, n und p ganze Zahlen im Bereich von etwa 2 bis 20 bedeuten und X- ein von einer anorganischen oder organischen Säure abgeleitetes Anion ist.

6. Zusammensetzung nach einem der Ansprüche 1 bis 5, **dadurch gekennzeichnet, dass** sie ferner mindestens einen ergänzenden grenzflächenaktiven Stoff enthält, der unter den anionischen grenzflächenaktiven Stoffen vom Phosphattyp, Sulfonattyp und/oder Sulfattyp, amphoteren grenzflächenaktiven Stoffen, nichtionischen grenzflächenaktiven Stoffen oder kationischen grenzflächenaktiven Stoffen oder deren Gemischen ausgewählt ist.

7. Zusammensetzung nach einem der Ansprüche 1 bis 6, **dadurch gekennzeichnet, dass** das Silicon in Konzentrationen im Bereich von 0,05 bis 10 Gew.-%, vorzugsweise 0,1 bis 5 Gew.-% und noch bevorzugter 0,5 bis 3 Gew.-%, bezogen auf das Gesamtgewicht der fertigen Zusammensetzung, enthalten ist.

8. Zusammensetzung nach einem der Ansprüche 1 bis 7, **dadurch gekennzeichnet, dass** der anionische grenzflächenaktive Stoff vom Carboxytyp in einer Konzentration im Bereich von 3 bis 50 Gew.-% und vorzugsweise 3 bis 20 Gew.-%, bezogen auf das Gesamtgewicht der Zusammensetzung, enthalten ist.

9. Zusammensetzung nach einem der Ansprüche 1 bis 8, **dadurch gekennzeichnet, dass** das kationische Polymer in einer Konzentration im Bereich von 0,005 bis 10 Gew.-%, vorzugsweise 0,01 bis 5 Gew.-% und noch bevorzugter 0,1 bis 3 Gew.-%, bezogen auf das Gesamtgewicht der fertigen Zusammensetzung, vorliegt.

10. Zusammensetzung nach einem der Ansprüche 1 bis 9, **dadurch gekennzeichnet, dass** die reinigenden grenzflächenaktiven Stoffe insgesamt 3 bis 50 Gew.-%, bezogen auf das Gesamtgewicht der Zusammensetzung, ausmachen.

11. Kosmetische Zusammensetzung nach einem der Ansprüche 1 bis 10, **dadurch gekennzeichnet, dass** sie ferner einen oder mehrere Zusatzstoffe enthält, die unter den kationischen grenzflächenaktiven Stoffe, anionischen Polymeren, nichtionischen Polymeren, kationischen Polymeren, amphoteren Polymeren, Proteinen, Proteinhydrolysaten, Aminosäuren, Ceramiden, Pseudoceramiden, Hydroxysäuren, Vitaminen, Panthenol, Hydratisierungsmitteln, Wirkstoffen gegen Haarausfall oder gegen Seborrhoe, Sonnenschutzfiltern, Radikalfängern für freie Radikale und deren Gemischen ausgewählt sind.

12. Verwendung der kosmetischen Zusammensetzung nach einem der Ansprüche 1 bis 11 für die Pflege und gleichzeitig zur Reinigung von Keratinsubstanzen, wie der Haare und der Haut.

13. Verfahren zur Reinigung und zum Konditionieren von Keratinsubstanzen, wie Haaren, das darin besteht, auf die feuchten Keratinsubstanzen eine Zusammensetzung nach einem der Ansprüche 1 bis 11 in einer wirksamen Menge aufzutragen und gegebenenfalls nach einer Einwirkzeit mit Wasser zu spülen.
